(19) Europäisches Patentamt
European Patent Office
Office européen des brevets

(11) Numéro de publication : **0 045 710**
**B1**

(12) # FASCICULE DE BREVET EUROPÉEN

(45) Date de publication du fascicule du brevet : **04.07.84**

(51) Int. Cl.³ : **C 07 C 85/11**, C 07 C 85/24, C 07 C 87/60

(21) Numéro de dépôt : **81420116.6**

(22) Date de dépôt : **30.07.81**

(54) **Procédé de préparation sélective d'anilines métachlorées.**

(30) Priorité : **01.08.80 FR 8017325**

(43) Date de publication de la demande : **10.02.82 Bulletin 82/06**

(45) Mention de la délivrance du brevet : **04.07.84 Bulletin 84/27**

(84) Etats contractants désignés : **AT BE CH DE FR GB IT LI LU NL SE**

(56) Documents cités :
**EP-A- 0 001 371**
**EP-A- 0 001 372**
**DE-A- 2 042 368**
**CHEMICAL ABSTRACTS, vol. 69, no. 7, 12 août 1968, page 2494; abrégé 26891r Columbus, Ohio, US P.V. MOKROUSOV et al.: "Effect of copper on the catalytic properties of a palladium catalysts"**

(73) Titulaire : **RHONE-POULENC AGROCHIMIE**
**14-20, rue Pierre Baizet**
**F-69009 Lyon (FR)**

(72) Inventeur : **Cordier, Georges**
**50 chemin des Hermières**
**F-69340 Francheville (FR)**
Inventeur : **Fouilloux, Pierre**
**22 bis avenue Général Leclerc**
**F-69300 Caluire (FR)**

(74) Mandataire : **Chrétien, François et al**
**RHONE-POULENC AGROCHIMIE BP 9163**
**F-69263 Lyon Cedex 1 (FR)**

**EP 0 045 710 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention se rapporte à un procédé de préparation d'anilines substituées par le chlore en position méta par action de l'hydrogène sur des composés aromatiques azotés plus fortement halogénés. Les anilines métachlorées sont des intermédiaires notamment pour la fabrication de matières actives phytosanitaires.

La préparation de chloranilines substituées en position méta par réaction de polychloranilines en milieu acide avec de l'hydrogène sous pression, en présence d'un catalyseur à base de métal noble, a été décrite dans le brevet français 2 298 531. Le procédé décrit nécessite toutefois l'utilisation de pressions élevées et de quantités très importantes d'acide chlorhydrique, ce qui pose de graves problèmes de corrosion.

La demande de brevet français 7 904 482, non publiée, décrit un procédé de préparation d'anilines substituées en méta par le chlore par hydrodéchloration en milieu acide dans des conditions particulières, la réaction étant, de plus, effectuée en présence, dans le milieu aqueux, de cations métalliques lourds. Ceci permet de travailler dans des conditions de température et de pression plus modérées.

La demanderesse a maintenant découvert qu'on peut, dans ce même but, utiliser comme catalyseur solide une association du métal noble et d'un métal lourd qui sera défini ci-après.

Plus particulièrement, la présente invention a pour objet un procédé de préparation d'anilines substituées en position méta par du chlore, par hydrogénation catalytique en phase liquide, à chaud et sous pression, en présence de métaux nobles du groupe VIII de la classification périodique, de dérivés benzéniques azotés et chlorés, de formule :

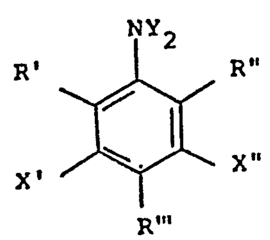

(I)

dans laquelle

Y représente l'atome d'hydrogène ou l'atome d'oxygène,

X′ et X″, identiques ou différents entre eux, représentent chacun un atome de chlore ou un radical alcoyle ou aryle ou aralcoyle ou alkoxyle ou aralkoxyle éventuellement substitué, au moins l'un des X′ et X″ étant obligatoirement un atome de chlore, l'un des X′ et X″ pouvant, de plus, être l'hydrogène,

R′, R″ et R‴, identiques ou différents entre eux, représentent chacun un atome de chlore ou un radical alcoyle ou aryle ou aralcoyle ou alcoxyle ou aryloxyle éventuellement substitué, l'un au moins de ces trois symboles représentant l'atome de chlore, au plus deux des R′, R″ ou R‴ pouvant, de plus, être l'hydrogène, caractérisé en ce que la réaction est effectuée en présence d'un cocatalyseur à base d'au moins un métal lourd appartenant à l'une des colonnes 1b à 5a de la classification périodique.

Comme métal lourd convenable, on peut citer en particulier le bismuth, le plomb, l'étain, le thallium, le mercure et l'argent. La demanderesse a notamment constaté que de bons résultats sont obtenus en utilisant l'argent.

La réaction s'effectue, comme il a été dit, en phase liquide ; en pratique, on opère avantageusement en présence d'un solvant minéral, liquide et inerte dans les conditions opératoires. Par solvant inerte, on entend un solvant ne réagissant pas chimiquement dans les conditions de la réaction. En fait, on préfère utiliser l'eau.

L'acidité du milieu réactionnel est généralement telle que le pH (en cas de milieu aqueux) est avantageusement inférieur à 1,5, de préférence inférieur à 1. La concentration en ions $H^+$ dans le milieu est généralement comprise entre 0,5 et 12 ion-g/l et de préférence entre 1 et 6 ion-g $H^+$/l. Des concentrations les plus élevées en acide peuvent être utilisées mais sans avantage important.

L'acidité du milieu réactionnel peut être obtenue par des acides forts minéraux tels que les acides sulfurique, phosphorique ou halohydriques, ou organiques ; mais l'on préfère l'utilisation des acides halohydriques et plus spécialement de l'acide chlorhydrique. De toute manière, étant donné la présence d'ions chlorure issus de la déshalogénation, on opère en fait en présence, au moins partiellement, d'acide chlorhydrique.

Le procédé selon l'invention s'effectue en phase liquide (hormis bien sûr le catalyseur à base de métal noble et de métal lourd). La phase liquide peut être homogène et constituer une solution : c'est là une modalité préférée, spécialement lorsque Y est l'atome d'oxygène dans la formule (I) ; une telle phase liquide contient donc les réactifs, les produits de réaction et le ou les solvants(s) éventuellement présent(s). Il est aussi possible d'opérer avec deux phases liquides.

La pression à laquelle s'effectue la réaction est généralement supérieure à 3 bars (pression relative)

et de préférence supérieure à 5 bars. Il n'y a pas de limite supérieure critique pour la pression mais pour des raisons d'ordre économique, il est généralement avantageux d'opérer à des pressions inférieures à 100 bars, les pressions inférieures à 20 bars étant préférées.

La température de réaction est généralement comprise entre 90 et 300 °C, de préférence entre 110 et 200 °C. Une température élevée peut, dans le cas où on utilise des acides relativement volatils, impliquer l'existence d'une pression partielle relativement importante pour les composés de la phase vapeur autres que l'hydrogène (par phase vapeur, on entend évidemment la phase vapeur surmontant le milieu liquide réactionnel). On choisit généralement les conditions opératoires de manière que la pression partielle d'hydrogène soit comprise entre 10 et 80 % de la pression totale (pression relative) et de préférence entre 30 et 60 %.

Les métaux nobles constituant la base des catalyseurs utilisés dans l'invention sont principalement des métaux du groupe VIII de la classification périodique tels que le ruthénium, le rhodium, le palladium, l'osmium, l'iridium et le platine ; le palladium est le métal préféré. Le métal peut être à l'état métallique ou à l'état de composé chimique ; généralement, on préfère que le métal soit mis en œuvre à l'état métallique car, dans les conditions opératoires, les composés ont tendance à être réduits à l'état métallique (degré d'oxydation = zéro).

Le catalyseur peut être supporté ou non supporté. Comme support du catalyseur, on peut utiliser tout support connu en soi pour supporter des catalyseurs, pourvu que ce support soit résistant à l'eau et aux acides ; comme support convenant plus particulièrement, on peut citer le noir de carbone, la silice, le sulfate de baryum ; le charbon actif est un support préféré. Le catalyseur ainsi que son support sont avantageusement sous forme finement divisée ; des surfaces spécifiques supérieures à 100 $m^2/g$ conviennent généralement bien.

La quantité de catalyseur mise en œuvre est telle que la proportion pondérale de métal noble du catalyseur par rapport au composé de formule (I) à traiter est généralement comprise entre 0,01 et 10 %, de préférence entre 0,1 et 5 %.

Les métaux lourds associés au métal noble utilisés dans l'invention ont, en pratique, un rôle de catalyseur favorisant la formation d'amines métachlorées. Ces métaux lourds peuvent être mis en œuvre à l'état métallique ou sous forme de composés solides. L'état métallique est préféré car, dans les conditions opératoires à caractère très réducteur, les composés ont tendance à être réduits à l'état métallique (degré d'oxydation = zéro). On a constaté que de bons résultats sont obtenus avec l'argent et l'étain. Les quantités de métal lourd par rapport au catalyseur à base de métal noble, exprimées en rapport molaire, sont le plus souvent comprises entre 0,1 et 10 et, de préférence, entre 1/3 et 3. Des rapports supérieurs peuvent être utilisés mais la proportion de métal noble devient faible, la réaction très lente et le procédé perd de son intérêt économique.

Comme composés de formule (I) susceptibles d'être traités par le procédé de l'invention, on peut citer de préférence :

— le dichloro-2,3 nitrobenzène et la dichloro-2,3 aniline ;
— le dichloro-2,5 nitrobenzène et la dichloro-2,5 aniline ;
— le dichloro-3,4 nitrobenzène et la dichloro-3,4 aniline ;
— le trichloro-2,3,4 nitrobenzène et la trichloro-2,3,4 aniline ;
— le trichloro-2,3,5 nitrobenzène et la trichloro-2,3,5 aniline ;
— le trichloro-2,3,6 nitrobenzène et la trichloro-2,3,6 aniline ;
— le trichloro-2,4,5 nitrobenzène et la trichloro-2,4,5 aniline ;
— le trichloro-3,4,5 nitrobenzène et la trichloro-3,4,5 aniline ;
— le tétrachloro-2,3,4,6 nitrobenzène et la tétrachloro-2,3,4,6 aniline ;
— le tétrachloro-2,3,4,5 nitrobenzène et la tétrachloro-2,3,4,5 aniline ;
— le tétrachloro-2,3,5,6 nitrobenzène et la tétrachloro-2,3,5,6 aniline ;
— le pentachloro-nitrobenzène et la pentachloroaniline ; mais aussi :
— le trichloro-4,5,6 méthyl-2 nitrobenzène et la trichloro-4,5,6 méthyl-2 aniline ;
— le dichloro-2,5 méthyl-4 nitrobenzène et la dichloro-2,5 méthyl-4 aniline ;
— le tétrachloro-2,3,5,6 méthyl-4 nitrobenzène et la tétrachloro-2,3,5,6 méthyl-4 aniline ;
— le dichloro-2,5 diméthyl-3,4 nitrobenzène et la dichloro-2,5 diméthyl-3,4 aniline ;
— le dichloro-2,5 éthyl-4 nitrobenzène et la dichloro-2,5 éthyl-4 aniline ;
— le dichloro-2,5 propyl-4 nitrobenzène et la dichloro-2,5 propyl-4 aniline ;
— le trichloro-3,4,6 benzyl-2 nitrobenzène et la trichloro-3,4,6 benzyl-2 aniline ;
— le dinitro-2,2′ hexachloro-3,5,6,3′,5′,6′ diphénylméthane et le diamino-2,2′ hexachloro-3,5,6,3′,5′,6′ diphénylméthane ;
— le nitro-2 trichloro-3,4,5 diphényle et l'amino-2 trichloro-3,4,5 diphényle ;
— le dinitro-4,4′ octachloro diphényle et le diamino-4,4′ octachloro diphényle ;
— le dichloro-4,5 méthoxy-2 nitrobenzène et la dichloro-4,5 méthoxy-2 aniline ;
— le dichloro-3,4 méthoxy-2 nitrobenzène et la dichloro-3,4 méthoxy-2 aniline ;
— le dichloro-3,6 méthoxy-2 nitrobenzène et la dichloro-3,6 méthoxy-2 aniline ;
— le dichloro-5,6 méthoxy-2 nitrobenzène et la dichloro-5,6 méthoxy-2 aniline ;
— le trichloro-3,4,6 méthoxy-2 nitrobenzène et la trichloro-3,4,6 méthoxy-2 aniline ;

— le trichloro-3,4,5 méthoxy-2 nitrobenzène et la trichloro-3,4,5 méthoxy-2 aniline ;
— le tétrachloro-3,4,5,6 méthoxy-2 nitrobenzène et la tétrachloro-3,4,5,6 méthoxy-2 aniline ;
— le dichloro-4,5 méthoxy-3 nitrobenzène et la dichloro-4,5 méthoxy-3 aniline ;
— le dichloro-5,6 méthoxy-3 nitrobenzène et la dichloro-5,6 méthoxy-3 aniline ;
— le dichloro-2,5 méthoxy-3 nitrobenzène et la dichloro-2,5 méthoxy-3 aniline ;
— le trichloro-4,5,6 méthoxy-3 nitrobenzène et la trichloro-4,5,6 méthoxy-3 aniline ;
— le tétrachloro-2,4,5,6 méthoxy-3 nitrobenzène et la tétrachloro-2,4,5,6 méthoxy-3 aniline ;
— le dichloro-2,3 méthoxy-4 nitrobenzène et la dichloro-2,3 méthoxy-4 aniline ;
— le dichloro-2,5 méthoxy-4 nitrobenzène et la dichloro-2,5 méthoxy-4 aniline ;
— le trichloro-2,3,6 méthoxy-4 nitrobenzène et la trichloro-2,3,6 méthoxy-4 aniline ;
— le trichloro-2,3,5 méthoxy-4 nitrobenzène et la trichloro-2,3,5 méthoxy-4 aniline ;
— le tétrachloro-2,3,5,6 méthoxy-4 nitrobenzène et la tétrachloro-2,3,5,6 méthoxy-4 aniline ;
— le dichloro-4,5 phénoxy-2 nitrobenzène et la dichloro-4,5 phénoxy-2 aniline ;
— le tétrachloro-3,4,5,6 phénoxy-2 nitrobenzène et la tétrachloro-3,4,5,6 phénoxy-2 aniline ;
— le tétrachloro-2,4,5,6 phénoxy-3 nitrobenzène et la tétrachloro-2,4,5,6 phénoxy-3 aniline ;
— le dichloro-2,5 phénoxy-4 nitrobenzène et la dichloro-2,5 phénoxy-4 aniline ;
— le tétrachloro-2,3,5,6 phénoxy-4 nitrobenzène et la tétrachloro-2,3,5,6 phénoxy-4 aniline.

Parmi les anilines substituées en position méta par l'atome de chlore et susceptibles d'être préparées par le procédé selon l'invention, on peut citer de préférence la métachloraniline et la dichloro-3,5 aniline mais aussi la chloro-5 méthyl-2 aniline ; la chloro-5 méthyl-3 aniline ; la chloro-3 méthyl-4 aniline ; la dichloro-3,5 méthyl-4 aniline ; la chloro-5 diméthyl-3,4 aniline ; la chloro-3 éthyl-4 aniline ; la chloro-3 benzyl-2 aniline ; le diamino-4,4' tétrachloro-2,6,2',6' diphényle ; la chloro-3 méthoxy-2 aniline ; la chloro-5 méthoxy-2 aniline ; la dichloro-3,5 méthoxy-2 aniline ; la chloro-3 méthoxy-4 aniline ; la chloro-5 méthoxy-3 aniline ; la dichloro-3,5 méthoxy-4 aniline ; la chloro-3 phénoxy-2 aniline ; la chloro-5 phénoxy-2 aniline ; la dichloro-3,5 phénoxy-2 aniline ; la dichloro-3,5 phénoxy-4 aniline.

Le procédé selon l'invention peut être effectué en continu ou en discontinu. En fin de réaction, le catalyseur et le cocatalyseur peuvent être séparés, le cas échéant, par filtration ou par des moyens équivalents tels que l'essorage ; l'aniline métachlorée préparée peut être séparée par tout moyen connu en soi par exemple par extraction à l'aide d'un solvant et/ou par distillation ; avant de procéder à cette séparation, il convient généralement de faire repasser l'aniline (salifiée en milieu acide) sous forme amine (non salifiée) par neutralisation ou alcalinisation à l'aide d'un agent alcalin.

Le procédé selon l'invention est très avantageux en raison de sa bonne sélectivité en amine métachlorée et des conditions relativement douces qu'il permet de réaliser.

Les exemples donnés à titre non limitatif, illustrent l'invention et montrent comment elle peut être mise en œuvre.

## Exemple 1

Dans un autoclave de 250 $cm^3$ revêtu intérieurement de tantale, on charge :

— 0,42 g de tétrachloro-2,3,4,5 aniline,

— 0,07 g d'un catalyseur constitué de palladium et d'argent déposés sur du charbon actif (surface spécifique du charbon actif : 1 100 $m^2/g$ ; teneur pondérale en palladium : 3 % ; teneur en argent : 2 %),

— 120 $cm^3$ d'une solution aqueuse d'acide chlorhydrique de concentration 4 moles/l.

L'autoclave est fermé, purgé d'abord à l'argon puis à l'hydrogène. On chauffe alors à 160 °C en laissant croître la pression autogène, puis, lorsque cette température est atteinte, on introduit l'hydrogène jusqu'à une pression totale (relative) de 13 bars dont 6 bars de pression partielle d'hydrogène.

On laisse réagir dans ces conditions pendant 2 heures. On refroidit ; la masse réactionnelle liquide est alcalinisée par une solution aqueuse de soude (NaOH) ; le catalyseur est séparé par filtration ; la dichloro-3,5 aniline est extraite de la phase aqueuse à l'aide de chlorure de méthylène ; la solution dans le chlorure de méthylène ainsi obtenue est séchée sur sulfate de sodium ; le solvant est évaporé sous vide.

Le taux de transformation de la tétrachloraniline a été de 100 %. Le rendement en dichloro-3,5 aniline obtenu est de 98 %. La teneur du milieu en argent est de 0,000 06 ion-g/l.

## Exemple 2

On opère comme à l'exemple 1 en remplaçant le catalyseur par un catalyseur constitué de palladium (teneur pondérale : 4 %) et l'argent (teneur pondérale : 1 %), le support étant le même que précédemment. La réaction dure 1 h 10 mn.

Dans ces conditions, on obtient la 3,5 dichloraniline avec un rendement de 87 % et la 3-chloraniline, comme sous-produit, avec un rendement de 10 %, le taux de transformation de la tétrachloraniline étant de 100 %.

**Revendications**

1. Procédé de préparation d'anilines substituées en position méta par du chlore, par hydrogénation catalytique en phase liquide, à chaud et sous pression, en présence de métaux nobles du groupe VIII de la classification périodique, de dérivés benzéniques azotés et chlorés, de formule :

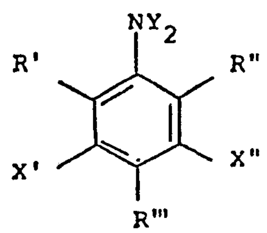

(I)

dans laquelle

Y représente l'atome d'hydrogène ou l'atome d'oxygène,

X' et X", identiques ou différents entre eux, représentent chacun un atome de chlore ou un radical alcoyle ou aryle ou aralcoyle ou alcoxyle ou aralcoxyle éventuellement substitué, au moins l'un des X' et X" étant obligatoirement un atome de chlore, l'un des X' et X" pouvant, de plus, être l'hydrogène,

R', R" et R"', identiques ou différents entre eux, représentent chacun un atome de chlore ou un radical alcoyle ou aryle ou aralcoyle ou alcoxyle ou aryloxyle éventuellement substitué, l'un au moins de ces trois symboles représentant l'atome de chlore, au plus deux des R', R" ou R"' pouvant, de plus, être l'hydrogène, caractérisé en ce que la réaction est effectuée en présence d'un cocatalyseur à base d'au moins un métal lourd appartenant à l'une des colonnes 1b à 5a de la classification périodique.

2. Procédé selon la revendication 1, caractérisé en ce que le métal lourd est choisi dans le groupe comprenant le bismuth, le plomb, l'étain, le thallium, le mercure et l'argent.

3. Procédé selon la revendication 1, caractérisé en ce que le métal lourd est l'argent.

4. Procédé selon l'une des revendications 1 à 3, caractérisé en ce que les quantités de métal lourd par rapport au catalyseur à base de métal noble, exprimées en rapport molaire, sont comprises entre 0,1 et 10 et, de préférence entre 1/3 et 3.

5. Procédé selon l'une des revendications 1 à 4, caractérisé en ce que R', R", R"', X', X", identiques ou différents entre eux, représentent l'atome d'hydrogène ou l'atome de chlore.

6. Procédé de préparation de dimétachloroanilines, éventuellement substituées, selon l'une des revendications 1 à 5, caractérisé en ce que X' et X" représentent l'atome de chlore.

7. Procédé de préparation de monométachloroanilines, éventuellement substituées, selon l'une des revendications 1 à 5, caractérisé en ce que un seul des deux radicaux X' et X" est l'atome de chlore.

8. Procédé de préparation de dichloro-3,5 aniline selon les revendications 1 à 6, caractérisé en ce que :

Y est l'atome d'hydrogène ou d'oxygène,

X' et X" sont l'atome de chlore,

R', R", R"' sont l'atome d'hydrogène ou l'atome de chlore, l'un d'entre eux au moins étant l'atome de chlore.

9. Procédé selon l'une des revendications 1 à 8, caractérisé en ce que le pH est inférieur à 1,5 et/ou que la concentration en ions $H^+$ du milieu réactionnel est comprise entre 0,5 et 12 ion-g/l.

10. Procédé selon la revendication 9, caractérisé en ce que le pH est inférieur à 1 et/ou que la concentration en ions $H^+$ du milieu réactionnel est comprise entre 1 et 6 ion-g/l.

11. Procédé selon l'une des revendications 1 à 10, caractérisé en ce que le milieu réactionnel est un milieu aqueux.

12. Procédé selon l'une des revendications 1 à 11, caractérisé en ce que le milieu réactionnel ne comporte qu'une phase liquide hormis le catalyseur à base de métal noble et de métal lourd.

13. Procédé selon l'une des revendications 1 à 12, caractérisé en ce que la pression totale est comprise entre 3 et 100 bars.

14. Procédé selon la revendication 13, caractérisé en ce que la pression totale est comprise entre 5 et 20 bars.

15. Procédé selon l'une des revendications 1 à 14, caractérisé en ce que la température est comprise entre 90 et 300 °C, de préférence entre 110 et 200 °C.

16. Procédé selon l'une des revendications 1 à 15, caractérisé en ce que la pression partielle d'hydrogène est comprise entre 10 et 80 % de la pression totale.

17. Procédé selon la revendication 16, caractérisé en ce que la pression partielle d'hydrogène est comprise entre 30 et 60 % de la pression totale.

18. Procédé selon l'une des revendications 1 à 17, caractérisé en ce que le catalyseur est le palladium.

19. Procédé selon l'une des revendications 1 à 18, caractérisé en ce que la proportion pondérale de métal noble par rapport au composé de formule (I) est comprise entre 0,01 et 10 %, de préférence entre 0,1 et 5 %.

**Claims**

1. A process for the preparation of anilines substituted in the meta-position by chlorine, by the catalytic hydrogenation, in the liquid phase, under the action of heat and under pressure, in the presence of noble metals from group VIII of the periodic classification, of nitrogen-containing and chlorine-containing benzene derivatives of the formula :

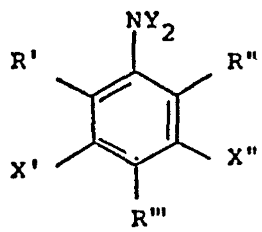

(I)

in which

Y represents the hydrogen atom or the oxygen atom,

X' and X", which are identical to or different from one another, each represent a chlorine atom or an optionally substituted alkyl, aryl, aralkyl, alkoxy or aralkoxy radical, at least one of the symbols X' and X" necessarily being a chlorine atom and it furthermore being possible for one of the symbols X' and X" to be hydrogen, and

R', R" and R"', which are identical to or different from one another, each represent a chlorine atom or an optionally substituted alkyl, aryl aralkyl, alkoxy or aryloxy radical, at least one of these three symbols representing the chlorine atom ant it being furthermore possible for at most two of the symbols R', R" or R"' to be hydrogen, characterized in that the reaction is carried out in the presence of a co-catalyst based on at least one heavy metal belonging to one of the groups Ib to Va of the periodic classification.

2. A process according to claim 1, wherein the heavy metal is chosen from the group comprising bismuth, lead, tin, thallium, mercury and silver.

3. A process according to claim 1, wherein the heavy metal is silver.

4. A process according to one of claims 1 to 3, wherein the amounts of heavy metal, relative to the catalyst based on a noble metal, and expressed as a molar ratio, are between 0.1 and 10 and preferably between 1/3 and 3.

5. A process according to one of claims 1 to 4, wherein R', R", R"', X' and X", which are identical to or different from one another, represent the hydrogen atom or the chlorine atom.

6. A process for the preparation of optionally substituted meta-dichloroanilines, according to one of claims 1 to 5, wherein X' and X" represent the chlorine atom.

7. A process for the preparation of optionally substituted meta-monochloroanilines, according to one of claims 1 to 5, wherein only one of the two radicals X' and X" is the chlorine atom.

8. A process for the preparation of 3,5-dichloroaniline, according to claims 1 to 6, wherein : Y is the hydrogen or oxygen atom, X' and X" are the chlorine atom and R', R" and R"' are the hydrogen atom or the chlorine atom, at least one of them being the chlorine atom.

9. A process according to one of claims 1 to 8, wherein the pH is less than 1.5 and/or the concentration of $H^+$ ions in the reaction medium is between 0.5 and 12 gram-ions/litre.

10. A process according to claim 9, wherein the pH is less than 1 and/or the concentration of $H^+$ ions in the reaction medium is between 1 and 6 gram-ions/litre.

11. A process according to one of claims 1 to 10, wherein the reaction medium is an aqueous medium.

12. A process according to one of claims 1 to 11, wherein the reaction medium only contains a liquid phase, except for the catalyst based on a noble metal and a heavy metal.

13. A process according to one of claims 1 to 12, wherein the total pressure is between 3 and 100 bars.

14. A process according to claim 13, wherein the total pressure is between 5 and 20 bars.

15. A process according to one of claims 1 to 14, wherein the temperature is between 90 and 300 °C, preferably between 110 and 200 °C.

16. A process according to one of claims 1 to 15, wherein the partial pressure of hydrogen is between 10 and 80 % of the total pressure.

17. A process according to claim 16, wherein the partial pressure of hydrogen is between 30 and 60 % of the total pressure.

18. A process according to one of claims 1 to 17, wherein the catalyst is palladium.

19. A process according to one of claims 1 to 18, wherein the proportion by weight of noble metal, relative to the compound of the formula (I), is between 0.01 and 10 %, preferably between 0.1 and 5 %.

**Ansprüche**

1. Verfahren zur Herstellung m-chlorsubstituierter Aniline durch katalytische Hydrierung von stickstoffhaltigen und chlorierten Benzolderivaten der Formel :

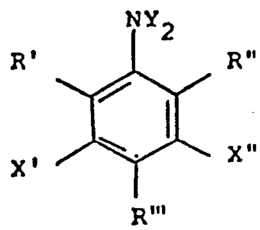

(I)

in der bedeuten

Y Wasserstoff oder Sauerstoff,

X′ und X″ zugleich oder unabhängig jeweils, Chlor oder Alkyl, Aryl, Aralkyl, Alkoxy oder Aralkoxy, die gegebenenfalls substituiert sein können, wobei dann, mindestens einer der Substituenten X′ und X″ zwingend Chlor ist, und einer der substituenten X′ und X″ ferner Wasserstoff bedeuten kann,

R′, R″ und R zugleich oder unabhängig jeweils Chlor oder Alkyl, Aryl, Aralkyl, Alkoxy oder Aryloxy, die gegebenenfalls substituiert sein können,

wobei mindestens einer dieser Substituenten Chlor darstellt und höchstens zwei der Substituenten R′, R″ bzw R ferner Wasserstoff bedeuten können, in flüssiger Phase in der Wärme und unter Druck in Gegenwart von Edelmetallen der Gruppe VIII des Periodensystems, dadurch gekennzeichnet, daß die Reaktion in Gegenwart eines Cokatalysators auf der Basis mindestens eines zu einer der Gruppen Ib bis Va des Periodensystems gehörenden Schwermetalls durchgeführt wird.

2. Verfahren nach Anspruch 1, dadurch gekennzeichnet, daß das Schwermetall unter Wismut, Blei, Zinn, Thallium, Quecksilber und Silber ausgewählt wird.

3. Verfahren nach Anspruch 1, dadurch gekennzeichnet, daß als Schwermetall Silber verwendet wird.

4. Verfahren nach einem der Ansprüche 1 bis 3, dadurch gekennzeichnet, daß das Molverhältnis von Schwermetall zu Katalysator auf der Basis des Edelmetalls zwischen 0,1 und 10, vorzugsweise zwischen 1/3 und 3, liegt.

5. Verfahren nach einem der Ansprüche 1 bis 4, dadurch gekennzeichnet, daß R′, R″, R‴, X′ und X″ zugleich oder unabhängig Wasserstoff oder Chlor bedeuten.

6. Verfahren zur Herstellung von gegebenenfalls substituierten Di-m-chloranilinen nach einem der Ansprüche 1 bis 5, dadurch gekennzeichnet, daß ein Benzolderivat der Formel I verwendet wird, in der X′ und X″ jeweils Chlor bedeuten.

7. Verfahren zur Herstellung gegebenenfalls substituierter Mono-m-chloraniline nach einem der Ansprüche 1 bis 6, dadurch gekennzeichnet, daß ein Benzolderivat der Formel I verwendet wird, in der nur einer der Substituenten X′ und X″ Chlor bedeutet.

8. Verfahren zur Herstellung von 3.5-Dichloranilin nach einem der Ansprüche 1 bis 6, dadurch gekennzeichnet, daß ein Benzolderivat der Formel I verwendet wird, in der Y Wasserstoff oder Sauerstoff, X′ und X″ jeweils Chlor und R′, R″ und R‴ Wasserstoff oder Chlor bedeuten, wobei mindestens einer dieser Substituenten Chlor ist.

9. Verfahren nach einem der Ansprüche 1 bis 8, dadurch gekennzeichnet, daß der pH-Wert unter 1,5 und/oder die $H^+$-Ionenkonzentration des Reaktionsgemischs zwischen 0,5 und 12 g-Ion/l beträgt.

10. Verfahren nach Anspruch 9, dadurch gekennzeichnet, daß der pH-Wert unter 1 und/oder die $H^+$-Ionenkonzentration des Reaktionsgemischs zwischen 1 und 6 g-Ion/l liegt.

11. Verfahren nach einem der Ansprüche 1 bis 10, dadurch gekennzeichnet, daß als Reaktionsmedium ein wäßriges Medium verwendet wird.

12. Verfahren nach einem der Ansprüche 1 bis 11, dadurch gekennzeichnet, daß ein Reaktionsmedium verwendet wird, das außer dem Katalysator auf der Basis des Edelmetalls und dem Schwermetall nur eine flüssige Phase aufweist.

13. Verfahren nach einem der Ansprüche 1 bis 12, dadurch gekennzeichnet, daß bei einem Gesamtdruck von 3 bis 100 bar verfahren wird.

14. Verfahren nach Anspruch 13, dadurch gekennzeichnet, daß bei einem Gesamtdruck von 5 bis 20 bar verfahren wird.

15. Verfahren nach einem der Ansprüche 1 bis 14, dadurch gekennzeichnet, daß bei einer Temperatur von 90 bis 300 °C vorzugsweise von 110 bis 200 °C.

16. Verfahren nach einem der Ansprüche 1 bis 15, dadurch gekennzeichnet, daß bei einem

Wasserstoffpartialdruck von 10 bis 80 % des Gesamtdrucks gearbeitet wird.

17. Verfahren nach Anspruch 16, dadurch gekennzeichnet, daß bei einem Wasserstoffpartialdruck von 30 bis 60 % des Gesamtdrucks gearbeitet wird.

18. Verfahren nach einem der Ansprüche 1 bis 17, dadurch gekennzeichnet, daß als Katalysor Palladium verwendet wird.

19. Verfahren nach einem der Ansprüche 1 bis 18, dadurch gekennzeichnet, daß der Gewichtsanteil des Edelmetalls 0,001 bis 10 %, vorzugsweise 0,1 bis 5 %, bezogen auf das Gewicht der Verbindung der Formel I, beträgt.